# EUROPEAN PATENT APPLICATION

(11) **EP 0 613 691 A1**
(43) Date of publication of application: **07.09.1994**
(21) Application number: 92917807.7
(22) Date of filing: 17.08.1992
(51) Int. Cl.: A61L 2/04, G02C 13/00

(54) **DEVICE FOR STERILIZING SOFT LENS BY BOILING**

(71) Applicant: TOMEI SANGYO KABUSHIKI KAISHA, Nagoya-shi, Aichi 451 (JP)
(72) Inventor: KAMIYA, Hideaki 11-8, Kamitsuchii 2-chome, Gifu 502 (JP)
(74) Representative: Türk, Gille, Hrabal, Leifert
(86) International application number: JP9201040
(87) International publication number: WO9404199

(57) **Abstract**

A device for sterilizing a soft lens by boiling, which is provided with: a body proper (2); a lid body (3) to be liquid-tightly sealed against the body proper (2); electrodes (13) for supplying electric current to solution (E) containing electrolyte in said body proper (2); and a gas permeating film (6) allowing gas and steam generated by electrolysis and by boiling of said solution (E) when current is supplied to pass through it toward the outside of the container (1) but not allowing liquid to pass; and, thus, the device is suitable for storing and carrying the lens, superior in heating efficiency for boiling, and capable of preventing abnormal heating.

## Description

### TECHNICAL FIELD

The present invention relates to a boiling device (to be simply called "device" hereinbelow) for sterilizing a soft contact lens. More particularly, it relates to a device for use in sterilizing, carrying and preserving a soft contact lens (to be simply called "lens" hereinbelow).

Conventionally, boiling for sterilizing a lens is conducted by accommodating the lens together with a preservative solution within a sealed container and heating the container from outside with a heating element such as an electric heater to boil the preservative solution therein (referred to as "prior art 1" hereinbelow).

Alternatively, there is known a device which uses a container having a usual opening on which a simple lid member is disposed (Japanese Unexamined Patent Application No. 110035/1989). In this device a lens is accommodated together with a cleaning solution within the container having a pair of electrodes therein, then electricity is made to flow through the cleaning solution to heat the solution owing to its own electrical resistance, thereby sterilizing the lens by boiling (referred to as "prior art 2" hereinbelow).

### BACKGROUND ART

In the case of prior art 1, the lens within the sealed container is indirectly heated through the container. Hence, excessive heating is needed. In addition vapor might be produced to raise the pressure within the sealed container, resulting in a danger. Furthermore, the temperature of the solution is raised to above 100°C, hence such a high temperature can deteriorate the lens rapidly.

In the case of prior art 2, moving the container might cause the lid member to come off from the opening thereof, which results in spill of the contents. Accordingly, the device is unsuitable to be carried and cannot be used except when it is placed on a table for sterilizing the lens therein.

If the container is sealed with the lid member, there would arise problems of an excessive rise in pressure and excessive heating, like prior art 1.

The present invention has been attained to overcome the foregoing problems. Thus, it is an object of the present invention to provide a compact boiling device suitable for carrying and preservation, of which the heating efficiency is increased and the internal pressure can be prevented from rising.

### DISCLOSURE OF THE INVENTION

According to the present invention, there is provided a device for boiling and sterilizing a soft contact lens, which is characterized by comprising a main body having an opening for accommodating a solution containing an electrolyte, a lid member for liquid-tightly closing the opening of the main body, a pair of electrodes for allowing electricity to flow through the solution, and ventilation means, which is impermeable with respect to liquid and permeable with respect to gas and vapor, for allowing escape of gas and vapor produced by electrolysis and boiling of the solution to the outside so as to mitigate a rise in internal pressure.

The device of the present invention is light and compact, and convenient for preservation and carrying of a lens because the solution containing an electrolyte never leaks out by virtue of the liquid-impermeable ventilation means. Further, the device is adapted to discharge gas and vapor produced by electrolysis and boiling of the solution containing an electrolyte without accumulation thereof so as to prevent the internal pressure from rising. This allows safe sterilization of a lens by boiling.

As described above, since the internal pressure of the device does not rise, the temperature of the solution never exceeds substantially 100°C, which greatly mitigates damage to a lens.

### BRIEF EXPLANATION OF THE DRAWINGS

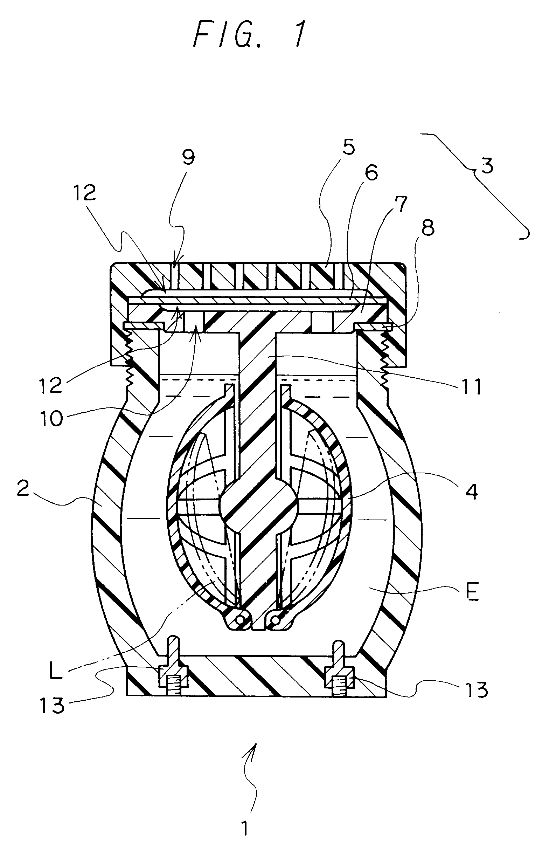

Fig. 1 is a sectional view showing an embodiment of a device according to the present invention.

### BEST MODE FOR CARRYING OUT THE INVENTION

Now, the device of the present invention will be described with reference to the attached drawing.

Referring to Fig. 1, denoted by numeral 1 is a device comprising a main body 2 which is substantially cylindrical and has a bottom, a lid member 3 removably mounted on the main body 2, and a basket 4 for holding a lens L in the main body 2.

The lid member 3 is integrally composed of a bottomed cylindrical cap 5 to be threadedly secured to the main body 2, and members fitted within the cap 5 in the following order: a circular ventilation film 6, a circular porous panel 7 and an annular gasket 8 which corresponds to the top end face of the main body 2. Threadedly securing such a lid member 3 to the main body 2 can make the device 1 liquid-tight.

The cap 5 is formed with a plurality of vent holes 9, while the ventilation film 6 has numerous micropores which permit vapor or gas to permeate and prevent liquid from permeating. The porous panel 7 is also formed with a plurality of vent holes 10 and provided with a holding member 11 downwardly extending centrally of the porous panel 7 for holding the basket 4. The cap 5 and the porous panel 7 are each formed with a recess in the surface facing opposite to the ventilation film 6, so that spaces 12 are defined on opposite sides of the ventilation film 6 when assembled into the lid member 3. The provision of such spaces 12 facilitates permeation of gas or vapor. Nevertheless, the ventilation film 6 might be directly sandwiched between the cap 5 and the porous panel 7 without providing the spaces 12, provided that the area of the ventilation film is large enough to pass therethrough a sufficient amount of gas or vapor.

In the bottom of the main body 2 are formed a pair of electrodes 13 which can be formed from any material usually employed therefor without particular limitation. Examples of such a material include a metal such as stainless steel, platinum, gold, copper, nickel and the like, a carbon rod plated or vapor-deposited with a metal such as gold and platinum, a synthetic resin treated with such plating or vapor deposition, and the like. Among these, in view of dissolution of electrode due to electrolytic reaction in each of the electrodes, preferable are a metal that is hard to ionize and dissolve when used for an electrode such as gold and platinum, and a synthetic resin plated or vapor-deposited with such a metal.

The ventilation film 6 is usually formed of a film having numerous micropores of about 0.1 to about 3.0 µm diameter and a thickness of about 75 to about 700 µm. It is preferable to form the micropores to at most 0.2 µm diameter because they will not permit microorganisms to pass therethrough, so that the solution having been sterilized is prevented from contamination. Examples of such a film include a Teflon film provided with a polypropylene net support, a microorganism filter imparted with a hydrophobic property, a hydrophobic high-precision filtration film such as of polyethylene and polypropylene, and a silicone film. Such a ventilation film can be bonded with a net-like reinforcement.

Next, the way of using the device 1 thus arranged is to be described.

A solution E containing an electrolyte is poured into the main body 2, and then the lid member 3 is threadedly secured to the main body 2 with the lens L held in the basket 4, so that the lens L is dipped in the solution E. In turn, voltage is applied across the electrodes 13 to allow current to flow through the solution E directly. As a result, the solution E generates heat because of its own electrical resistance, then boils. Hence, the lens L is sterilized and cleaned.

In response to application of voltage, the solution E is immediately electrolyzed to generate gas, then boiled to generate vapor. Such gas and vapor are discharged passing through the vent holes 10 of the porous panel 7, ventilation film 6 and the vent holes 9 of the cap 5 sequentially. Therefore, the internal pressure of the main body 2 barely rises. Hence, the temperature of the solution E will not exceed substantially 100°C, so that the lens L is never damaged by heat.

In the device of the present invention, the shape and the size of each of the main body, lid member, basket and the like are not particularly limited. It is possible to use the main body shaped substantially prismatic, spherical or hemispherical, and the lid member shaped to correspond to the main body. It is also possible to make the device small for accommodating therein only a pair of lenses or to make it large enough to accommodate therein two pairs of lenses or more and sterilize them simultaneously.

The basket is not limited to that supported by the holding member 11 secured to the porous panel 7 as shown in Fig. 1. It can be held by a holding member disposed in the main body or can be put into the main body without using a holding member.

The size, mounted state and the way of mounting of the ventilation film are not limited to those shown in Fig. 1, unless the ventilation film is completely dipped in the solution E and always in contact with it.

Although the electrodes 13 are mounted on the bottom of the main body 2 in the device shown in Fig. 1, the location of the electrodes 13 is not limited to the bottom of the main body 2, and they can be located anywhere as far as they always contact the solution E. For example, they can be mounted on the inner side wall of the main body 2, on the basket 4 or on the holding member 11 supporting the basket 4. Otherwise, the electrodes 13 can be formed to have a rod-like shape downwardly extending from the lid member 3. However, it is preferable to dispose them in a location the lowest possible for promoting convection of the solution E.

In addition, the electrodes 13 does not necessarily project inwardly from the main body 2, as in the device 1 shown in Fig. 1. They might be coplanar with the inside surface of the main body 2. Besides, the number of pair of electrodes is not limited to one. It is possible to provide a plurality of pairs of electrodes.

The device of the present invention will never leak a solution containing an electrolyte to the outside, and is hence suitable for preservation and carrying of a lens. In contrast, the device is adapted to make the internal pressure escape to the outside, thereby preventing a lens from being excessively pressurized and heated. Further, the device enjoys a good heating efficiency, resulting in a shortened heating time.

## Claims

1. A device for boiling and sterilizing a soft contact lens, which comprises a main body having an opening for accommodating a solution containing an electrolyte, a lid member for liquid-tightly closing the opening of the main body, a pair of electrodes for allowing electricity to flow through the solution, and ventilation means, which is impermeable with respect to liquid and permeable with respect to gas and vapor, for allowing escape of gas and vapor produced by electrolysis and boiling of the solution to the outside so as to mitigate a rise in internal pressure.
